# NOUVEAU FASCICULE DE BREVET EUROPEEN

(11) **EP 0 689 419 B2**
(45) Date de publication et mention de la décision concernant l'opposition: **28.06.2000**
(45) Mention de la délivrance du brevet: 11.12.1996
(21) Numéro de dépôt: 94909962.6
(22) Date de dépôt: 15.03.1994
(51) Int. Cl.: A61K 7/48, A61K 7/06

(54) **UTILISATION EN COSMETIQUE OU EN APPLICATION TOPIQUE D'UNE DISPERSION AQUEUSE A BASE D'ORGANOPOLYSILOXANES NON VOLATILS ET D'UN POLYMERE RETICULE DE CHLORURE DE METHACRYLOYLOXYETHYL TRIMETHYLAMMONIUM, DE TYPE HOMOPOLYMERE OU COPOLYMERE AVEC L'ACRYLAMIDE**
VERWENDUNG EINER WÄSSRIGEN DISPERSION VON NICHTFLÜCHTIGEN ORGANOPOLYSILOXANEN UND EINEM VERNETZTEN METHACRYLOYLOXYETHYL TRIMETHYLAMMONIUMAMMONIUMCHLORIDE-HOMO- ODER CO-POLYMER MIT ACRYLAMID
COSMETIC OR TOPICAL USE OF AN AQUEOUS DISPERSION BASED ON NON-VOLATILE ORGANOPOLYSILOXANES AND AN ACRYLAMIDE OR HOMOPOLYMERIC COPOLYMER TYPE CROSS-LINKED METHACRYLOYLOXYETHYL TRIMETHYLAMMONIUM CHLORIDE POLYMER

(30) Priorité: 16.03.1993 FR 9302987
(43) Date de publication de la demande: 03.01.1996
(73) Titulaire: L'OREAL, 75008 Paris (FR)
(72) Inventeur: CAUWET, Danièle, F-75011 Paris (FR); DUBIEF, Claude, F-78150 Le Chesnay (FR)
(74) Mandataire: Casalonga, Axel
(86) Numéro de dépôt international: FR9400275
(87) Numéro de publication internationale: WO9421224

(56) Documents cités:
- EP-A- 0 181 773
- EP-A- 0 219 830
- EP-A- 0 424 260
- EP-A- 0 524 434
- WO-A-86/02546
- WO-A-92/10162
- WO-A-94/02176
- US-A- 4 960 764
- Allied Colloids data sheet on Salcare SC92 published February 1992
- "Liquid Grade Rheology Modifiers for Cosmetic Products" by N. Ambrose, Allied Colloids, printed version of a lecture held at the IN-COSMETICS Conference in Frankfurt on 4 March 1992

## Description

L'invention concerne l'utilisation en cosmétique ou en application topique, d'une dispersion aqueuse à base d'organopolysiloxanes non volatils et d'un polymère réticulé de chlorure de méthacryloyloxyéthyl triméthylammonium, de type homopolymère ou copolymère avec l'acrylamide.

On utilise déjà des huiles siliconées en cosmétique comme lubrifiant dans les compositions de traitement des cheveux et de la peau. Il s'agit principalement de polydiméthylsiloxanes.

Afin d'apporter de la douceur aux cheveux ou à la peau, ou encore de faciliter le démêlage des cheveux, on utilise depuis longtemps des polymères ou des tensio-actifs cationiques. Les composés cationiques présentent l'inconvénient, après applications répétées, d'alourdir la chevelure en lui donnant un aspect poisseux ou de produire un effet collant sur la peau.

Le document EP-A-0 424 260 décrit des dispersions aqueuses pour le traitement cosmétique de la peau et des cheveux contenant l'association d'une silicone et d'un copolymère d'acrylate d'ammonium/acrylamide réticulé.

Le document EP-A-0 524 434 décrit des shampooings conditionneurs contenant un tensio-actif anionique et un copolymère réticulé de chlorure de méthacryloyloxy éthyl triméthoxylammonium et d'acrylamide. Ce document n'envisage pas l'utilisation dans les compositions d'un organopolysiloxane non-volatil.

La demanderesse a découvert, d'une manière surprenante, que l'utilisation d'une dispersion aqueuse à base d'organopolysiloxanes non volatils et d'un polymère réticulé de chlorure de méthacryloyloxyéthyl triméthylammonium, de type homopolymère ou copolymère avec l'acrylamide pour le traitement des cheveux, permet d'obtenir des cheveux brillants, soyeux, légers, dont les propriétés de démêlage, de douceur et de tenue sont sensiblement améliorées. En outre, le temps de séchage des cheveux est plus court.

L'utilisation de cette dispersion aqueuse dans le traitement de la peau, permet également de conférer à celle-ci un toucher doux sans effet collant.

Les dispersions aqueuses utilisées en cosmétique ou en application topique, selon la présente invention, se répartissent beaucoup plus facilement sur la peau et sur les cheveux que les compositions de l'art antérieur à base de composés cationiques.

La demanderesse a découvert également que les compositions cosmétiques sous forme de dispersion aqueuse, selon la présente invention, étaient remarquablement stables, que leurs propriétés cosmétiques se conservaient même après plusieurs applications successives.

Un objet de l'invention est donc constitué par l'utilisation dans le traitement cosmétique des cheveux ou de la peau ou en application topique, d'une dispersion aqueuse contenant au moins un organopolysiloxane défini ci-après et d'un polymère réticulé de chlorure de méthacryloyloxyéthyl triméthylammonium, de type homopolymère ou copolymère avec l'acrylamide.

Un autre objet de l'invention concerne des compositions cosmétiques ou dermatologiques pour le traitement des cheveux ou de la peau, sous forme de dispersions aqueuses.

Un autre objet de l'invention concerne des procédés de traitement cosmétique des cheveux ou de la peau, mettant en oeuvre ces compositions, selon l'application désirée.

D'autres objets de l'invention apparaîtront à la lumière de la description et des exemples qui suivent.

La présente invention a pour objet principal l'utilisation pour le traitement cosmétique des cheveux ou de la peau ou en application topique, d'une dispersion aqueuse, caractérisée par le fait que celle-ci contient au moins dans un milieu aqueux cosmétiquement ou physiologiquement acceptable, un polymère réticulé de chlorure de méthacryloyloxyéthyl triméthylammonium, de type homopolymère ou copolymère avec l'acrylamide et un organopolysiloxane non volatil choisi parmi les polyalkylsiloxanes, les polyarylsiloxanes, les polyalkylarylsiloxanes, les gommes et résines de silicone et les polysiloxanes organomodifiés à l'exclusion des polysiloxanes portant des groupements polyéthylèneoxy et/ou polypropylèneoxy, ou carboxylates ou bisulfites.

Toutefois sont exclues de la présente invention les compositions dont il est fait mention dans le présente revendication 1 et divulguées par le document WO94/02176 dont il est tenu compte conformément à l'article 54 (3) CBE.

Les organopolysiloxanes utilisés dans les dispersions selon la présente invention, sont des huiles non volatiles d'organopolysiloxanes ou des solutions organiques de gomme ou de résine d'organosiloxanes ou encore des émulsions ou microémulsions renfermant ces organopolysiloxanes.

Parmi les polyalkylsiloxanes, on peut citer principalement les polydiméthylsiloxanes linéaires : soit,
- à groupements terminaux triméthylsilyle, comme par exemple, et à titre non limitatif, les huiles SILBIONE de la série 70047 commercialisées par RHONE POULENC : l'huile 47 V 500.000 de RHONE POULENC ou certaines Viscasil de la GENERAL ELECTRIC, ou
- à groupements terminaux trihydroxysilyle, telles que les huiles de la série 48 V de RHONE POULENC.

Dans cette classe de polyalkylsiloxanes, on peut également mentionner les polyalkylsiloxanes vendus par la Société GOLDSCHMIDT sous les dénominations ABILWAX 9800 et ABILWAX 9801, qui sont des polyalkyl(C₁-C₂₀)siloxanes.

Parmi les polyalkylarylsiloxanes, on peut citer les polydiméthylphénylsiloxanes, les polydiméthyldiphénylsiloxanes linéaires et/ou ramifiés, de viscosité 10⁻⁵ à 5.10⁻² m²/s à 25°C, tels que, par exemple :
- l'huile RHODORSIL 763 de RHONE POULENC,
- les huiles SILBIONE de la série 70641 de RHONE POULENC, telles que les huiles SILBIONE 70641 V 30 et 70641 V 200 de RHONE POULENC,
- le produit DC 556 Cosmetic Grad Fluid de DOW CORNING,
- les silicones des séries PK de BAYER, telles que la PK20,
- les silicones des séries PN, PH de BAYER, comme les PN 1000 et PH 1000,
- certaines huiles des séries SF de GENERAL ELECTRIC, telles que les SF 1250, SF 1265, SF 1154, SF 1023,
- l'huile 618 V 25000 de RHONE POULENC.

Les gommes de silicone, conformes à la présente invention, sont des polydiorganosiloxanes de forte masse moléculaire comprise entre 200 000 et 1 000 000, utilisés seuls ou en mélange dans un solvant choisi parmi les huiles polydiméthylsiloxanes (PDMS), les huiles polyphénylméthylsiloxanes (PPMS), les isoparaffines, le chlorure de méthylène, le pentane, le dodécane, le tridécane, le tétradécane ou leurs mélanges.

On cite, par exemple, les composés suivants :
- polydiméthylsiloxane éventuellement hydroxylé en bout de chaîne,
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)].

On peut citer, par exemple, à titre non limitatif, les mélanges suivants :
1/ les mélanges formés à partir d'un polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit Q2 1401 vendu par la Société DOW CORNING;
2/ les mélanges formés à partir d'une gomme polydiméthylsiloxane avec une silicone cyclique, tel que le produit SF 1214 SILICONE FLUID de GENERAL ELECTRIC, qui est une gomme SE 30 de PM 500.000 solubilisée dans la SF 1202 SILICONE FLUID (décaméthylcyclopentasiloxane);
3/ les mélanges de deux PDMS de viscosités différentes, notamment d'une gomme PDMS et d'une huile PDMS, tels que les produits SF 1236 et CF 1241 de la Société GENERAL ELECTRIC. Le produit SF 1236 est le mélange d'une gomme SE 30 définie ci-dessus d'une viscosité de 20 m²/s et d'une huile SF 96 d'une viscosité de 5.10⁻⁶ m²/s (15% de gomme SE 30 et 85% d'huile SF 96).

Le produit CF 1241 est le mélange d'une gomme SE 30 (33%) et d'une PDMS (67%) de viscosité 10⁻³ m²/s.

Les résines d'organopolysiloxanes utilisables conformément à l'invention, sont des systèmes siloxaniques réticulés renfermant les unités :
R₂SiO_{2/2}, RSiO_{3/2} et SiO_{4/2} dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.

Parmi ces résines, on peut citer le produit vendu sous la dénomination DOW CORNING 593 ou ceux vendus sous les dénominations SILICONE FLUID SS 4230 et SS 4267 par la Société GENERAL ELECTRIC et qui sont des "diméthyl/triméthylpolysiloxane".

Les silicones organomodifiées, conformes à la présente invention, sont des silicones telles que définies précédemment, comportant dans leur structure générale, un ou plusieurs groupements organofonctionnels directement fixés sur la chaîne siloxanique ou fixés par l'intermédiaire d'un radical hydrocarboné.

On cite, par exemple, les silicones comportant :
a) des groupements fluorés tels que des trifluoroalkyles telles que, par exemple, celles vendues par la Société GENERAL ELECTRIC sous les dénominations "FF.150 Fluorosilicone Fluid" ou par la Société SHIN ETSU sous les dénominations X-22-819; X-22-820; X-22-821; X-22-822;
b) des groupements hydroxyacylamino telles que celles décrites dans la demande de brevet européen EPA 0342834 et en particulier la silicone vendue par la Société DOW CORNING sous la dénomination Q2-8413;
c) des groupements thiols comme dans les silicones X 2-8360 de la DOW CORNING ou les GP 72A et GP 71 de GENESEE;
d) des groupements aminés substitués ou non, comme dans la GP4 SILICONE FLUID de GENESEE, la GP 7100 de GENESEE, la Q2 8220 de DOW CORNING, l'AFL 40 d'UNION CARBIDE ou la silicone dénommée "Amodiméthicone" dans le dictionnaire CTFA;
e) des groupements hydroxylés, comme les polyorganosiloxanes à fonction hydroxyalkyle et notamment γ-hydroxypropyle, décrits dans la demande de brevet en France n° FR-85 16334;
f) des groupements alcoxylés comme dans la Silicone copolymer F 755 de SWS SILICONES et les produits ABILWAX 2428, ABILWAX 2434, ABILWAX 2440 de l a Société GOLDSCHMIDT;
g) des groupements acyloxyalkyle, comme par exemple les polyorganopolysiloxanes décrits dans la demande de brevet français n° 88 17433, et en particulier des groupements γ-stéaroyloxypropyle;
h) des groupements ammonium quaternaire, comme dans les produits X2 81 08 et X2 81 09, le produit ABIL K 3270 de la Société GOLDSCHMIDT;
i) des groupements amphotères ou bétaïniques, tels que dans le produit vendu par la Société GOLDSCHMIDT sous la dénomination ABIL B 9950;

Les polyorganosiloxanes utilisés selon l'invention, sont présents dans la dispersion aqueuse dans une proportion comprise entre 0,2 et 50% en poids, de préférence entre 1 et 30% en poids, par rapport au poids total de la dispersion.

Les polymères réticulés de chlorure de méthacryloyloxyéthyl triméthylammonium utilisés selon l'invention, sont plus particulièrement choisis parmi les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylènebis acrylamide.

On utilise plus particulièrement un copolymère réticulé acrylamide/chlorure de méthacryloyloxyéthyl triméthylammonium (20/80 en poids) sous forme de dispersion contenant 50% en poids dudit copolymère dans de l'huile minérale. Cette dispersion est commercialisée sous le nom de SALCARE SC92 par la Société ALLIED COLLOIDS. On utilise également un homopolymère réticulé du chlorure de méthacryloyloxyéthyl triméthylammonium contenant environ 50% en poids de l'homopolymère dans de l'huile minérale. Cette dispersion est commercialisée sous le nom de SALCARE SC95 par la Société ALLIED COLLOIDS.

Les polymères réticulés tels que définis ci-dessus, sont présents dans les dispersions aqueuses de l'invention à des concentrations en matière active comprises entre 0,05 et 10% en poids et de préférence entre 0,1 et 6% en poids par rapport au poids total de la dispersion.

Parmi les polyorganosiloxanes, on préfère selon l'invention, utiliser des silicones organomodifiées par des groupements thiols, des groupements aminés substitués ou non, des groupements fluorés et des gommes et résines de silicone.

Parmi les polymères réticulés, on préfère, selon l'invention, utiliser un copolymère réticulé d'acrylamide et de chlorure de méthacryloyloxyéthyl triméthylammonium (20/80).

Les dispersions conformes à l'invention peuvent contenir en plus des adjuvants habituellement utilisés en cosmétique ou en dermatologie, tels que des parfums, des colorants, des conservateurs, des stabilisants, des agents séquestrants, des cires, des agents nacrants, des huiles végétales, animales ou synthétiques, des perfluoropolyéthers, des céramides, des filtres solaires, des anti-radicaux libres, des agents tensio-actifs anioniques, non-ioniques, amphotères ou cationiques, des polymères, des protéines, des stabilisateurs de mousse, des propulseurs, suivant l'application envisagée.

Les compositions cosmétiques destinées au traitement des cheveux, conformes à l'invention, peuvent être utilisées en particulier comme shampooing, comme produit à rincer, à appliquer avant ou après shampooing, avant, pendant ou après coloration ou décoloration, avant ou après permanente ou défrisage ou en lotion intrapermanente ou comme produit coiffant non rincé, tel que dans des lotions de mises en plis ou de brushing.

Les compositions cosmétiques conformes à la présente invention destinées au traitement et aux soins de la peau, peuvent être sous forme de produit pour le bain ou la douche, de produit bronzant, de produit pour le rasage, de lotion parfumée, de crème ou de lait pour le soin de la peau ou des compositions antisolaires.

Lorsque les compositions selon l'invention contiennent des tensioactifs, ceux-ci sont présents en une concentration comprise entre 5 et 30% lorsqu'il s'agit de shampooings ou de gels douche, et en une proportion inférieure à 5% en poids par rapport au poids total de la composition lorsqu'il s'agit de compositions rincées à appliquer après shampooings ou de compositions non rincées.

Les compositions conformes à la présente invention peuvent être appliquées en dermatologie. Elles contiennent, en une quantité efficace, une substance active sur le plan dermatologique, telle que par exemple la vitamine A, les caroténoïdes, les protéines, les pigments naturels, des rétinoïdes, des dépigmentants, des substances anti-séborrhéiques ou antiacnéiques, des anti-inflammatoires, des anti-pelliculaires.

Les compositions cosmétiques ou dermatologiques, selon la présente invention, présentent un pH compris entre 3 et 10 et de préférence entre 3 et 7. Ce pH peut être ajusté par des agents alcalinisants ou acidifiants habituellement utilisés en cosmétique et en dermatologie.

Un procédé de traitement cosmétique des cheveux, selon l'invention, consiste à appliquer les compositions telles que définies ci-dessus sur les cheveux, suivant l'usage envisagé (shampooing, traitement à rincer, traitement de coiffage sans rinçage), sans qu'il soit nécessaire d'observer un temps de pose et à rincer éventuellement.

Un procédé de traitement cosmétique de la peau, selon l'invention, consiste à appliquer sur celle-ci une composition telle que définie ci-dessus, selon l'usage envisagé (bain, douche, produits bronzants, produits pour le rasage, lotions parfumées, crèmes ou laits de soin) et à rincer éventuellement.

Les exemples qui suivent sont destinés à illustrer la présente invention, sans pour autant présenter un caractère limitatif.

### EXEMPLE 1

On prépare un après-shampooing de composition suivante :

| | |
|---|---|
| - Dispersion dans l'huile minérale de copolymère réticulé acrylamide/chlorure de méthacryloyloxyéthyl triméthylammonium, vendu à 50% en copolymère sous la dénomination "SALCARE SC 92" par la Société ALLIED COLLOIDS | 0,5 g en copolymère |
| - Polydiméthyldiphénylsiloxane (90/10) α,ω-disilanol, vendu sous la dénomination "HUILE 618 V 25000" par la Société RHONE POULENC | 10 g |
| - Conservateur qs | |
| - pH spontané = 5 | |
| - Eau déminéralisée qsp | 100 g |

### EXEMPLE 2

On prépare un après-shampooing de composition suivante :

| | |
|---|---|
| - Dispersion dans l'huile minérale de copolymère réticulé acrylamide/chlorure de méthacryloyloxyéthyl triméthylammonium, vendu à 50% en copolymère sous la dénomination "SALCARE SC 92" par la Société ALLIED COLLOIDS | 0,5 g en copolymère |
| - Polyméthyltrifluoropropylsiloxane vendu sous la dénomination "FF 150-10M" par la Société GENERAL ELECTRIC | 1 g |
| - Conservateur qs | |
| - pH spontané = 4,5 | |
| - Eau déminéralisée qsp | 100 g |

### EXEMPLE 3

On prépare un après-shampooing de composition suivante :

| | |
|---|---|
| - Dispersion dans l'huile minérale de copolymère réticulé acrylamide/chlorure de méthacryloyloxyéthyl triméthylammonium, vendu à 50% en copolymère sous la dénomination "SALCARE SC 92" par la Société ALLIED COLLOIDS | 0,5 g en copolymère |
| - Polydiméthyl/méthyltrifluoropropylsiloxane vendu sous la dénomination "X-22-820" par la Société SHIN ETSU | 1 g |
| - Conservateur qs | |
| - pH spontané = 4 | |
| - Eau déminéralisée qsp | 100 g |

### EXEMPLE 4

On prépare un après-shampooing de composition suivante :

| | |
|---|---|
| - SALCARE SC 92 | 0,75 g en copolymère |
| - PDMS vendu sous la dénomination "HUILE 47 V 500.000" par la Société RHONE POULENC | 0,75 g |
| - Méthylglucose oxyéthyléné à 20 moles d'oxyde d'éthylène | 1,05 g |
| - Conservateurs qs | |
| - pH spontané = 3,8 | |
| - Eau déminéralisée qsp | 100 g |

### EXEMPLE 5

On prépare un après-shampooing de composition suivante :

| | |
|---|---|
| - SALCARE SC 92 | 1,96 g en copolymère |
| - PDMS vendu sous la dénomination "HUILE 47 V 500.000" par la Société RHONE POULENC | 0,25 g |
| - Huile minérale | 5 g |
| - Conservateurs | 0,1 g |
| - pH spontané = 3,6 | |
| - Eau déminéralisée qsp | 100 g |

### EXEMPLE 6

On prépare un shampooing de composition suivante :

| | |
|---|---|
| - SALCARE SC 92 | 0,2 g MA |
| - PDMS vendu sous la dénomination "HUILE 47 V 500.000" par la Société RHONE POULENC | 2 g |
| - Lauryl éther sulfate de sodium à 2,2 moles d'oxyde d'éthylène en solution aqueuse à 28% de MA | 16,8 g MA |
| - Cocoylbétaïne en solution aqueuse à 32% de MA | 2,6 g MA |
| - Ether de cétyle et d'hydroxy-2-cétyl-stéaryle/alcool cétylique | 2,5 g |
| - Monoisopropanolamide d'acide de coprah | 1,5 g |
| - Conservateur, parfum qs | |
| - NaOH qs pH=6,5 | |
| - Eau qsp | 100 g |

### EXEMPLE 7

On a préparé un produit solaire de composition suivante :

| | |
|---|---|
| - SALCARE SC 95 | 1,5 g MA |
| - PDMS (Silbione 70047 V 300 RHONE POULENC) | 5 g |
| - Polydécène hydrogéné (Ethylflo 364 NF d' Ethyl Corp.) | 5 g |
| - Paraméthoxycinnamate d'éthyl-2 hexyle (Parsol MCX de Givaudan) | 4 g |
| - Triéthanolamine qs pH=7 | |
| - Conservateur qs | |
| - Eau qsp | 100 g |

## Revendications

1. Utilisation en cosmétique ou en application cosmétique topique d'une dispersion aqueuse, caractérisée par le fait que ladite dispersion contient au moins, dans un milieu aqueux cosmétiquement ou physiologiquement acceptable, un organopolysiloxane non volatil choisi parmi les polyalkylsiloxanes, les polyarylsiloxanes, les polyalkylarylsiloxanes, les polysiloxanes modifiés ou non, les gommes et résines de silicone, les polysiloxanes organomodifiés à l'exception des polysiloxanes ponant des groupements polyéthylènoxy et/ou polypropylènoxy ou carboxylates ou bisulfites et un polymère réticulé de chlorure de méthacryloyloxyéthyl triméthylammonium de type homopolymère ou copolymère avec l'acrylamide, et à l'exclusion des dispersions ayant les compositions suivantes :
| | A | B | C | D | E |
|---|---|---|---|---|---|
| Eau purifiée | qs pour 100 | qs pour 100 | qs pour 100 | qs pour 100 | qs pour 100 |
| Ibuprofène | - | 2,0 | - | - | - |
| PPG-14 Butyl Ether | 8,0 | - | 8,0 | - | - |
| Urée | - | - | - | 10,0 | - |
| Polyquaternium 32 et Huile minérale¹ | 2,5 | 4,0 | - | 4,0 | 3 |
| Diméthylaminoéthyl méthacrylate quaternisé Méthyle réticulé et Huile Minérale³ | - | - | 1,5 | - | - |
| Polyoxyéthylène 10 Cétyl Ether | 0,75 | 0,75 | - | 0,75 | 0,75 |
| Steareth-21 | - | - | 0,5 | - | - |
| Acide salicylique | 2,0 | - | 2,0 | - | - |
| Dihydroxyacétone | - | - | - | - | 3,0 |
| Alcool stéarylique | 0,75 | 0,75 | 1,5 | 0,75 | 0,75 |
| Alcool cétylique | 0,75 | 0,75 | 1,5 | 0,75 | 0,75 |
| 85% 5 cs diméthyl fluide/15% Diméthiconol² | 0,5 | 0,5 | 0,5 | 0,5 | 0,5 |
| Octényl succinate d'amidon d'aluminium | 0,5 | 0,5 | - | 0,5 | 0,5 |
| Diméthicone 200 | - | - | 0,6 | - | - |
| Glycérol | - | - | 3,0 | - | 2,0 |
| Acide citrique | - | - | 0,7 | - | - |
| Citrate de sodium | - | - | 0,3 | - | - |
| | | | | | |
|---|---|---|---|---|---|
| ¹Salcare SC92 disponible auprès de Allied Colloids (Suffolk, VA) | | | | | |
| ²Dow Q2-1403 fluid disponible auprès de Dow Corning | | | | | |
| ³Salcare SC95 disponible auprès de Allied Colloids. | | | | | |
dans les compositions A, B, D, E le polyquaternium 32 et l'huile minérale peuvent être replacés par un diméthylaminoéthyl méthacrylate quaternisé méthyle réticulé et huile minérale (polyquaternium 37 et huile minérale et PPG-1 trideceth-6), disponible sous l'appellation Salcare SC95 auprès de Allied Colloids et dans la composition C le diméthylaminoéthyl méthacrylate quaternisé methyle réticulé et huile minérale peuvent être replacés par le polyquaternium 32 et huile minérale.

2. Utilisation selon la revendication 1, caractérisée par le fait que l'organopolysiloxane est choisi parmi:
A/ les polyakyl(C₁-C₂₀)siloxanes; les polydiméthylsiloxanes linéaires, à groupements terminaux triméthylsilyle et les polydiméthylsiloxanes linéaires à groupements terminaux trihydroxysilyle;
B/ les polydiméthylphénylsiloxanes, les polydiméthyldiphénylsiloxanes linéaires et/ou ramifiés, de viscosité 10⁻⁵ à 5.10⁻² m²/s à 25°C;
C/ les gommes de masse moléculaire comprise entre 200 000 et 1 000 000, utilisées seules ou sous forme de mélange dans un solvant, choisies dans le groupe constitué par les composés suivants:
- polydiméthylsiloxane éventuellement hydroxylé en bout de chaîne,
- poly[(diméthylsiloxane)/(méthylvinylsiloxane)],
- poly[(diméthylsiloxane)/(diphénylsiloxane)],
- poly[(diméthylsiloxane)/(phénylméthylsiloxane)],
- poly[(diméthylsiloxane)/(diphénylsiloxane)/(méthylvinylsiloxane)];
et les mélanges suivants
- les mélanges formés à partir d'un polydiméthylsiloxane hydroxylé en bout de chaîne et d'un polydirnéthylsiloxane cyclique;
- les mélanges formés à partir d'une gomme polydiméthylsiloxane et d'une silicone cyclique; - les mélanges de deux polydiméthylsiloxanes de viscosités différentes;
D/ les résines d'organopolysiloxanes renfermant les unités R2SiO2/2, RSiO3/2 et SiO4/2 dans lesquelles R représente un groupement hydrocarboné possédant 1 a 6 atomes de carbone ou un groupement phényle.

3. Utilisation selon la revendication 1, caractérisée par le fait que l'organopolysiloxane comporte dans sa structure générale un ou plusieurs groupement(s) organofonctionnel(s) directement fixé(s) sur la chaîne siloxanique ou fixé(s) par l'intermédiaire d'un radical hydrocarboné, et qu'il est choisi parmi les polyorganosiloxanes comportant:
a) des groupes fluorés;
b) des groupes hydroxyacylamino;
c) des groupements thiol;
d) des groupes aminés substitués ou non;
e) des groupements hydroxyalkyle;
f) des groupements alcoxylés;
g) des groupements acyloxyalkyle;
h) des groupements ammonium quaternaire;
i) des groupements amphotères ou bétaïniques;

4. Utilisation selon l'une quelconque des revendications 1 à 3, caractérisée par le fait que le copolymère d'acrylamide/chlorure de méthacryloyloxyéthyl triméthylammonium est obtenu par copolymérisation de l'acrylamide et du diméthylaminoéthyl méthacrylate quaternisé par le chlorure de méthyle et que l'homopolymère est obtenu par homopolymérisation du diméthylaminoéthylméthacrylate quaternisé par le chlorure de méthyle, la polymérisation étant suivie d'une réticulation par un composé à insaturation oléfinique, en particulier le méthylène-bis acrylamide.

5. Utilisation selon l'une quelconque des revendications 1 à 4, caractérisée par le fait que l'on met en oeuvre le copolymère réticulé acrylamide/chlorure de méthacryloyloxyéthyl triméthylammonium (20/80 en poids) sous forme de dispersion contenant 50% en poids dudit copolymère dans de l'huile minérale.

6. Utilisation selon l'une quelconque des revendications 1 à 4, caractérisée par le fait que l'on met en oeuvre l'homopolymère réticulé de chlorure de méthacryloyloxyéthyl triméthylammonium sous forme de dispersion dans l'huile minérale.

7. Utilisation selon l'une quelconque des revendications 1 à 6, caractérisée par le fait que l'organopolysiloxane est présent dans des proportions comprises entre 0,2 et 50% en poids par rapport au poids total de la dispersion et le polymère réticulé est présent dans des proportions comprises entre 0,05 et 10% en poids.

8. Composition cosmétique sous forme de dispersion aqueuse destinée au traitement des cheveux ou de la peau, caractérisée par le fait que la dispersion aqueuse est telle que définie dans l'une quelconque des revendications 1 à 7.

9. Composition selon la revendication 8, caractérisée par le fait qu'elle contient en plus des adjuvants habituellement utilisés en cosmétique, choisis parmi les parfums, les colorants, les conservateurs, les stabilisants, des cires ou des huiles végétales, animales ou synthétiques, les agents nacrants, des protéines, des agents de conditionnement, les agents tensio-actifs anioniques, non-ioniques, amphotères ou cationiques, les séquestrants, les stabilisateurs de mousse, des polymères, des filtres solaires, des propulseurs, des perfluoropolyéthers, des céramides, des anti-radicaux libres.

10. Composition selon l'une des revendications 8 ou 9, caractérisée par le fait qu'elle présente un pH compris entre 3 et 10 et de préférence entre 3 et 7.

11. Composition selon l'une quelconque des revendications 8 à 10, destinée au traitement des cheveux, caractérisée par le fait qu'elle se présente sous forme de shampooing, de produit à rincer, a appliquer avant ou après shampooing, avant, pendant ou après coloration ou décoloration, avant ou après permanente ou défrisage, en lotion intrapermanente, ou comme produits de coiffage non rincés.

12. Composition selon l'une quelconque des revendications 8 à 10, destinée au traitement de la peau, caractérisée par le fait qu'elle se présente comme produit de bain ou de douche, de produit bronzant, de composition anti-solaire, de produit de rasage, de crème ou de lait pour le soin ou de lotion parfumée.

13. Composition selon l'une quelconque des revendications 8 à 11, caractérisée par le fait qu'elle se présente sous forme de shampooing ou de produit de bain ou de douche, contenant de 5 à 30% en poids de tensio-actifs par rapport au poids total de la composition.

14. Composition selon l'une quelconque des revendications 8 à 11, caractérisée par le fait qu'elle se présente sous forme de produit à rincer à appliquer après shampooing ou de produit de coiffage non rincé et qu'elle contient des tensio-actifs dans une proportion inférieure a 5% en poids par rapport au poids total de la composition.

15. Procédé de traitement cosmétique des cheveux, caractérisé par le fait que l'on applique sur ceux-ci au moins une composition telle que définie dans la revendication 11.

16. Procédé de traitement cosmétique de la peau, caractérisé par le fait que, l'on applique sur celle-ci une composition telle que définie dans la revendication 12.

17. Composition dermatologique sous forme de dispersion aqueuse, caractérisée par le fait que la dispersion aqueuse est telle que définie dans l'une quelconque des revendications 1 à 14 et qu'elle contient au moins une substance active sur le plan dermatologique dans une quantité efficace.

## Claims

1. Use in cosmetics or in topical application of an aqueous dispersion, characterized in that the said dispersion contains at least, in a cosmetically or physiologically acceptable aqueous medium, one non-volatile organopolysiloxane chosen from polyalkylsiloxanes, polyarylsiloxanes, polyalkylarylsiloxanes, modified or unmodified polysiloxanes, silicone gums and resins or organomodified polysiloxanes, with the exception of polysiloxanes carrying polyethyleneoxy and/or polypropyleneoxy or carboxylate or bisulphite groups, and one crosslinked methacryloyloxyethyltrimethylammonium chloride polymer of homopolymer or copolymer with acrylamide type and with the exclusion of dispersions having the following compositions:
| | A | B | C | D | E |
|---|---|---|---|---|---|
| Purified water | qs 100 | qs 100 | qs 100 | qs 100 | qs 100 |
| Ibuprofen | - | 2.0 | - | - | - |
| PPG-14 Butyl Ether | 8.0 | - | 8.0 | - | - |
| Urea | - | - | - | 10.0 | - |
| Polyquaternium-32 and Mineral oil¹ | 2.5 | 4.0 | - | 4.0 | 3 |
| Crosslinked methyl-quaternized dimethylaminoethyl methacrylate and Mineral oil³ | - | - | 1.5 | - | - |
| Polyoxyethylene 10 Cetyl Ether | 0.75 | 0.75 | - | 0.75 | 0.75 |
| Steareth-21 | - | - | 0.5 | - | - |
| Salicylic acid | 2.0 | - | 2.0 | - | - |
| Dihydroxyacetone | - | - | - | - | 3.0 |
| Stearyl alcohol | 0.75 | 0.75 | 1.5 | 0.75 | 0.75 |
| Cetyl alcohol | 0.75 | 0.75 | 1.5 | 0.75 | 0.75 |
| 85% 5 cs dimethyl fluid/15% Dimethiconol² | 0.5 | 0.5 | 0.5 | 0.5 | 0.5 |
| Aluminium starch octenylsuccinate | 0.5 | 0.5 | - | 0.5 | 0.5 |
| Dimethicone 200 | - | - | 0.6 | - | - |
| Glycerol | - | - | 3.0 | - | 2.0 |
| Citric acid | - | - | 0.7 | - | - |
| Sodium citrate | - | - | 0.3 | - | - |
| | | | | | |
|---|---|---|---|---|---|
| ¹Salcare SC92 available from Allied Colloids (Suffolk, VA) | | | | | |
| ²Dow Q2-1403 fluid available from Dow Corning | | | | | |
| ³Salcare SC95 available from Allied Colloids | | | | | |
In compositions A, B, D and E, the polyquaternium-32 and the mineral oil can be replaced with a crosslinked methyl-quaternized dimethylaminoethyl methacrylate and mineral oil (polyquaternium-37 and mineral oil and PPG-1 trideceth-6), available under the name Salcare SC95 from Allied Colloids, and in composition C the crosslinked methyl-quaternized dimethylammonium methacrylate and mineral oil can be replaced with polyquaternium-32 and mineral oil.

2. Use according to Claim 1, characterized in that the organopolysiloxane is chosen from:
A) polyalkyl(C₁-C₂₀)siloxanes; linear polydimethylsiloxanes containing end trimethylsilyl groups and linear polydimethylsiloxanes containing end trihydroxysilyl groups;
B) linear and/or branched polydimethylphenylsiloxanes or polydimethyldiphenylsiloxanes, with a viscosity of 10⁻⁵ to 5.10⁻² m²/s at 25°C;
C) gums with a molecular mass of between 200,000 and 1,000,000, used alone or in the form of a mixture in a solvent, chosen from the group composed of the following compounds:
- polydimethylsiloxane, optionally hydroxylated at the chain end,
- poly[(dimethylsiloxane)/(methylvinylsiloxane)],
- poly[(dimethylsiloxane)/(diphenylsiloxane)],
- poly[(dimethylsiloxane)/(phenylmethylsiloxane)],
- poly[(dimethylsiloxane)/(diphenylsiloxane)/(methylvinylsiloxane)];
and the following mixtures;
- the mixtures formed from a polydimethylsiloxane hydroxylated at the chain end and from a cyclic polydimethylsiloxane;
- the mixtures formed from a polydimethylsiloxane gum and from a cyclic silicone;
- the mixtures of two polydimethylsiloxanes of different viscosities;
D) the organopolysiloxane resins containing the R₂SiO_{2/2}, RSiO_{3/2} and SiO_{4/2} units, in which R represents a hydrocarbon group having 1 to 6 carbon atoms or a phenyl group.

3. Use according to Claim 1, characterized in that the organopolysiloxane contains, in its general structure, one or a number of organofunctional group(s) directly attached to the siloxane chain or attached via a hydrocarbon radical and in that it is chosen from the polyorganosiloxanes containing:
a) fluorinated groups;
b) hydroxyacylamino groups;
c) thiol groups;
d) substituted or unsubstituted amino groups;
e) hydroxyalkyl groups;
f) alkoxylated groups;
g) acyloxyalkyl groups;
h) quaternary ammonium groups;
i) amphoteric or betaine groups;

4. Use according to any one of Claims 1 to 3, characterized in that the acrylamide-methacryloyloxyethyltrimethylammonium chloride copolymer is obtained by copolymerization of acrylamide and of dimethylaminoethyl methacrylate quaternized by methyl chloride and in that the homopolymer is obtained by homopolymerization of dimethylaminoethyl methacrylate quaternized by methyl chloride, the polymerization being followed by crosslinking by a compound possessing olefinic unsaturation, in particular methylenebisacrylamide.

5. Use according to any one of Claims 1 to 4, characterized in that the crosslinked acrylamidemethacryloyloxyethyltrimethylammonium chloride (20/80 by weight) copolymer is used in the form of a dispersion containing 50% by weight of the said copolymer in mineral oil.

6. Use according to any one of Claims 1 to 4, characterized in that the crosslinked methacryloyloxyethyltrimethylammonium chloride homopolymer is used in the form of a dispersion in mineral oil.

7. Use according to any one of Claims 1 to 6, characterized in that the organopolysiloxane is present in proportions of between 0.2 and 50% by weight with respect to the total weight of the dispersion and the crosslinked polymer is present in proportions of between 0.05 and 10% by weight.

8. Cosmetic composition in the form of an aqueous dispersion intended for the treatment of hair or of the skin, characterized in that the aqueous dispersion is as defined in any one of Claims 1 to 7.

9. Composition according to Claim 8, characterized in that it additionally contains adjuvants commonly used in cosmetics chosen from fragrances, dyes, preservatives, stabilizers, waxes or vegetable, animal or synthetic oils, pearlescent agents, proteins, conditioning agents, anionic, nonionic, amphoteric or cationic surfactants, sequestering agents, foam stabilizers, polymers, sunscreens, propellants, perfluoropolyethers, ceramides or agents for combating free radicals.

10. Composition according to either of Claims 8 and 9, characterized in that it has a pH of between 3 and 10 and preferably between 3 and 7.

11. Composition according to any one of Claims 8 to 10 intended for the treatment of hair, characterized in that it is provided in the form of a shampoo, of a product to be rinsed out, to be applied before or after a shampoo, before, during or after dyeing or bleaching, before or after a permanent wave or hair straightening, in a lotion to be applied during the perming operation, or as nonrinsed styling products.

12. Composition according to any one of Claims 8 to 10 intended for the treatment of the skin, characterized in that it is provided as a bath or shower product, of [sic] a tanning product, of [sic] an antisun composition, of [sic] a shaving product, of [sic] a care cream or milk or of [sic] a scented lotion.

13. Composition according to any one of Claims 8 to 11, characterized in that it is provided in the form of a shampoo or of a bath or shower product containing from 5 to 30% by weight of surfactants with respect to the total weight of the composition.

14. Composition according to any one of Claims 8 to 11, characterized in that it is provided in the form of a product to be rinsed out to be applied after a shampoo or of a nonrinsed styling product and in that it contains surfactants in a proportion of less than 5% by weight with respect to the total weight of the composition.

15. Process for the cosmetic treatment of hair, characterized in that at least one composition as defined in Claim 11 is applied to the hair.

16. Process for the cosmetic treatment of the skin, characterized in that a composition as defined in Claim 12 is applied to the skin.

17. Dermatological composition in the form of an aqueous dispersion, characterized in that the aqueous dispersion is as defined in any one of Claims 1 to 14 and in that it contains, in an effective amount, at least one substance which is active at the dermatological level.

## Patentansprüche

1. In der Kosmetik oder einer topischen kosmetischen Aufbringung vorgesehene Verwendung einer wässrigen Dispersion,
dadurch **gekennzeichnet,** dass
die genannte Dispersion mindestens, in einem kosmetisch oder physiologisch zulässigen Milieu, ein nicht-flüchtiges Organopolysiloxan, ausgewählt aus Polyalkylsiloxanen, Polyarylsiloxanen, Polyalkylarylsiloxanen, gegebenenfalls modifizierten Polysiloxanen, Gummi- und Harzprodukten aus Silikon und aus organomodifizierten Siloxanen, ausgenommen Polysiloxane, die Polyethylenoxy- und/oder Polypropylenoxy- oder Carboxylat- oder Bisulfit-Gruppierungen aufweisen, sowie ein vernetztes Polymer aus Methacryloyloxyethyltrimethylammoniumchlorid vom Typ eines Homopolymer oder eines entsprechenden mit Acrylamid gebildeten Copolymer enthält, wobei Dispersionen mit den folgenden Zusammensetzungen ausgeschlossen sind:
| | A | B | C | D | E |
|---|---|---|---|---|---|
| gereinigtes Wasser | Menge auf 100 | Menge auf 100 | Menge auf 100 | Menge auf 100 | Menge auf 100 |
| Ibuprofen | - | 2,0 | - | - | - |
| PPG-14-Butylether | 8,0 | - | 8,0 | - | - |
| Harnstoff | - | - | - | 10,0 | - |
| Polyquaternium 32 und Mineralöl¹ | 2,5 | 4,0 | - | 4,0 | 3 |
| vernetztes quaterniertes Dimethylaminoethylmethylmethacrylat und Mineralöl³ | - | - | 1,5 | - | - |
| Polyoxyethylen-10-Cetylether | 0,75 | 0,75 | - | 0,75 | 0,75 |
| Steareth-21 | - | - | 0,5 | - | - |
| Salicylsäure | 2,0 | - | 2,0 | - | - |
| Dihydroxyaceton | - | - | - | - | 3,0 |
| Stearylalkohol | 0,75 | 0,75 | 1,5 | 0,75 | 0,75 |
| Cetylalkohol | 0,75 | 0,75 | 1,5 | 0,75 | 0,75 |
| 85% 5 cs Dimethyl-Flüssigkeit / 15% Dimethiconol ² | 0,5 | 0,5 | 0,5 | 0,5 | 0,5 |
| Aluminium-Stärkeoctenylsuccinat | 0,5 | 0,5 | - | 0,5 | 0,5 |
| Dimethicon 200 | - | - | 0,6 | - | - |
| Glycerin | - | - | 3,0 | - | 2,0 |
| Zitronensäure | - | - | 0,7 | - | - |
| Natriumzitrat | - | - | 0,3 | - | - |
| | | | | | |
|---|---|---|---|---|---|
| ¹ Salcare SC92, erhältlich von Allied Colloids (Suffolk, VA) | | | | | |
| ² Dow Q2-1403-Flüssigkeit, erhältlich von Dow Corning | | | | | |
| ²Salcare SC95, erhätlich von Allied Colloids. | | | | | |
In den Zusammensetzungen A, B, D, E kann das Polyquaternium 32 mit dem Mineralöl durch ein vernetztes quaterniertes Dimethylaminoethylmethylmethacrylat mit Mineralöl (Poyquaternium 37 und Mineralöl und PPG-1-Trideceth-6), erhältlich unter der Bezeichnung Salcare SC95 von Allied Colloids, und in der Zusammensetzung C kann das vernetzte quaternierte Dimethylaminoethylmethylmethacrylat mit Mineralöl durch das Polyquaternium 32 und dem Mineralöl ersetzt werden.

2. Verwendung gemäß Anspruch 1,
dadurch **gekennzeichnet,** daß
das Organopolysiloxan ausgewählt ist aus:
A) Poly-(C₁₋₂₀)-alkylsiloxanen, linearen Polydimethylsiloxanen mit endständigen Trimethylsilygruppen und linearen Polydimethylsiloxanen mit endständigen Trihydroxysilylgruppen;
B) linearen und/oder verzweigten Polydimethylphenylsiloxanen und Polydimethyldiphenylsiloxanen einer Viskosität bei 25°C von 10⁻⁵ bis 5 x 10⁻² m²/s;
C) Gummiprodukten einer Molekularmasse von 200.000 bis 1.000.000, die alleine oder in Form einer Mischung in einem Lösungsmitel verwendet werden, das aus der Gruppe ausgewählt ist, die sich aus folgenden Verbindungen zusammensetzt:
- Polydimethylsiloxan, gegebenenfalls hydroxyliert am Kettenende,
- Poly((dimethylsiloxan)/(methylvinylsiloxan)),
- Poly((dimethylsiloxan)/(diphenylsiloxan)),
- Poly((dimethylsiloxan)/(phenylmethylsiloxan)),
- Poly((dimethylsiloxan)/(diphenylsiloxan)/(methylvinylsiloxan)); sowie aus den folgenden Mischungen:
- Mischungen, die aus einem am Kettenende hydroxylierten Polydimethylsiloxan und einem zyklischen Polydimethylsiloxan gebildet sind;
- Mischungen, die aus einem Polydimethylsiloxan-Gummi und einem zyklischen Silikon gebildet sind;
- Mischungen aus zwei Polydimethylsiloxanen unterschiedlicher Viskositäten;
D) Harzen von Organopolysiloxanen, enthaltend die Einheiten R₂SiO_{2/2}, RSiO_{3/2} und SiO_{4/2}, in denen R eine Kohlenwasserstoffgruppe mit 1 bis 6 Kohlenstoffatomen oder eine Phenylgruppe darstellt.

3. Verwendung gemäß Anspruch 1,
dadurch **gekennzeichnet,** daß
das Organopolysiloxan in seiner allgemeinen Struktur ein oder mehrere organofunktionelle Gruppierungen aufweist, die direkt oder über das Zwischenglied eines Kohlenwasserstoffrests an die Siloxankete gebunden sind, und aus Polyorganosiloxanen ausgewählt ist, die enthalten:
a) fluorierte Gruppen;
b) Hydroxyacylaminogruppen;
c) Thiol-Gruppierungen;
d) Amingruppen, substituiert oder nicht;
e) Hydroxyalkylgruppierungen;
f) alkoxylierte Gruppierungen;
g) Acyloxyalkylgruppierungen;
h) quaternäre Ammoniumgruppierungen;
i) amphotere oder beatinische Gruppierungen.

4. Verwendung gemäß einem der Ansprüche 1 bis 3,
dadurch **gekennzeichnet,** daß
das Copolymer aus Acrylamid/Methacryloyloxyethyltrimethylammoniumchlorid durch Copolymerisation von Acrylamid und von mit Methylchlorid quaterniertem Dimethylaminoethylmethacrylat und das Homopolymer durch Homopolymerisation des mit Methylchlorid quaternierten Dimethylaminoethylmethacrylats erhältlich sind, wobei auf die Polymerisation eine Vernetzung mit einer olefinisch ungesättigten Verbindung, insbesondere mit Methylenbisacrylamid, erfolgt.

5. Verwendung gemäß einem der Ansprüche 1 bis 4,
dadurch **gekennzeichnet,** daß
man das vernetzte Copolymer aus Acrylamid/Methacryloyloxyethyltrimethylammoniumchlorid (20/80, gewichtsbezogen) in Form einer Dispersion einsetzt, die 50 Gew.% des genannten Copolymer in Mineralöl enthält.

6. Verwendung gemäß einem der Ansprüche 1 bis 4,
dadurch **gekennzeichnet,** daß
man das vernetzte Homopolymer aus Methacryloyloxyethyltrimethylammoniumchlorid in Form einer in Mineralöl gebildeten Dispersion einsetzt.

7. Verwendung gemäß einem der Ansprüche 1 bis 6,
dadurch **gekennzeichnet,** daß
das Organopolysiloxan in Mengenanteilen von 0,2 bis 50 Gew.%, bezogen auf das Gesamtgewicht der Dispersion, und das vernetzte Polymer in Mengenanteilen von 0,05 bis 10 Gew.% vorhanden sind.

8. Kosmetische Zusammensetzung in Form einer wässrigen Dispersion zur Behandlung der Haare oder der Haut,
dadurch **gekennzeichnet,** daß
die wässrige Dispersion die in jedem der Ansprüche 1 bis 7 definierte ist.

9. Zusammensetzung gemäß Anspruch 8,
dadurch **gekennzeichnet,** daß
sie zusätzlich Hilfsstoffe enthält, die gewöhnlich in der Kosmetik verwendet werden und aus Parfüm-Produkten, Farbstoffen, Konservierungsstoffen, Stabilisiermitteln, pflanzlichen, tierischen oder synthetischen Wachsen oder Ölen, Perlmuttmitteln, Proteinen, Konditioniermitteln, anionischen, nicht-ionischen, amphoteren oder kationischen oberflächenaktiven Mitteln, Sequestriermitteln, Schaumstabilisatoren, Polymeren, Sonnenfilterstoffen, Treibmitteln, Perfluorpolyethern, Ceramiden und aus Abfangmitteln für freie Radikale ausgewählt sind.

10. Zusammensetzung gemäß einem der Ansprüche 8 oder 9,
dadurch **gekennzeichnet,** daß
sie einen pH-Wert von 3 bis 10 und vorzugsweise von 3 bis 7 aufweist.

11. Zusammensetzung gemäß einem der Ansprüche 8 bis 10 zur Behandlung der Haare,
dadurch **gekennzeichnet,** daß
sie in Form eines Shampoo, Produkts zur Spülung, zur Aufbringung vor oder nach dem Schamponieren, vor, bei oder nach Färbung oder Entfärbng, vor oder nach Dauerwelle oder Entfrisieren, als eine Lotion zwischen einer Dauerwelle oder als nicht zur Spülung vorgesehenes Frisierprodukt vorliegt.

12. Zusammensetzung gemäß einem der Ansprüche 8 bis 10 zur Behandlung der Haut,
dadurch **gekennzeichnet,** daß
sie als Produkt für Bad oder Dusche, Bräunungsprodukt, Sonnenschutzmittelzusammensetzung, Rasurprodukt, Creme oder Milch zur Pflege oder parfümierte Lotion vorliegt.

13. Zusammensetzung gemäß einem der Ansprüche 8 bis 11,
dadurch **gekennzeichnet,** daß
sie in Form eines Shammpoo oder eines Produkts für Bad oder Dusche vorliegt, welche 5 bis 30 Gew.% oberflächenatkives Mittel enthalten, bezogen auf das Gesamtgewicht der Zusammensetzung.

14. Zusammensetzung gemäß einem der Ansprüche 8 bis 11,
dadurch **gekennzeichnet,** daß
sie in Form eines Produkts zur Spülung zur Aufbringung nach Schamponierung oder eines nicht zur Spülung vorgesehenen Frisierprodukts vorliegt und oberflächenaktive Mittel in einem Mengenanteil von weniger als 5 Gew.% enthält, bezogen auf das Gesamtgewicht der Zusammensetzung.

15. Verfahren zur kosmetischen Behandlung der Haare,
dadurch **gekennzeichnet,** daß
man auf diese mindestens eine in Anspruch 11 definierte Zusammensetzung aufbringt.

16. Verfahren zur kosmetischen Behandlung der Haut,
dadurch **gekennzeichnet,** daß
man auf sie eine in Anspruch 12 definierte Zusammensetzung aufbringt.

17. Dermatologische Zusammensetzung in Form einer wässrigen Dispersion,
dadurch **gekennzeichnet,** daß
die wässrige Dispersion eine in jedem der Ansprüche 1 bis 14 definierte ist und mindestens eine auf dem dermatologischen Plan wirksame Substanz in einer wirksamen Menge enthält.
